# EUROPEAN PATENT APPLICATION

(11) **EP 4 549 556 A1**
(43) Date of publication of application: **07.05.2025**
(21) Application number: 23207226.4
(22) Date of filing: 31.10.2023
(51) Int. Cl.: C12N 5/079

(54) **METHOD FOR GENERATION OF ENDOCANNABINOID RECEPTOR GENE KNOCKOUT MICROGLIA CELLS**

(71) Applicant: Vilnius University, 01513 Vilnius (LT)
(72) Inventor: Baltriukiene, Daiva, Vilnius (LT); Iesmantaite, Monika, Vilnius (LT); Baltrukonyte, Emilija, Vilnius (LT); Bukelskiene, Virginija, Vilnius (LT)
(74) Representative: AAA Law

(57) **Abstract**

The present invention relates to a method for generation of the immortalized microglia cells with knockout of endocannabinoid receptor gene. The cells are useful model for developing strategies to treat neurodegenerative diseases.

## Description

### FIELD OF THE INVENTION

The present invention relates to a production of a model to investigate the role of endocannabinoid receptors in microglia in neuroinflammatory processes, neurodegenerative diseases, or other neurological disorders.

### BACKGROUND OF THE INVENTION

Microglia cells are a specialized population of macrophages that function as cell of the innate immune system in the central nervous system (CNS). These cells comprise 10-15% of all cells in the human brain and play an important role in CNS development as well as in maintaining proper neuronal function throughout the entire life of an individual. Microglia cells, through their interaction with neurons, other cells, and molecules, perform dynamic, constant monitoring of their surrounding microenvironment. This enables microglia to detect and respond to various infections, tissue damage, or homeostatic perturbations. However, the main function of microglial cells include phagocytosis and the provision of trophic support as well as the continuous active regulation of synapse formation, elimination, and plasticity in the brain (Cser6p *et al.,* 2020; Lee *et al.,* 2021). Microglia can take on a range of pro- or anti-inflammatory phenotypes to maintain homeostasis. Neuroinflammation is characterised by persistent pro-inflammatory activation of microglia, which releases toxic cytokines that cause widespread damage to the brain (Young and Denovan-Wright, 2021). In this context, microglia cells can not only provide tissue repair but can also contribute to neuronal damage by releasing harmful substances, including inflammatory cytokines. The endocannabinoid system modulates the release of inflammatory factors such as cytokines and could represent a functional link between microglia and neuroinflammatory processes (De Meij, 2021). The CB1 receptor, one of two cannabinoid receptors, is abundantly expressed in the central and peripheral nervous system. Its expression is especially high in brain regions associated with the regulation of emotion, mood, cognition and stress responses, suggesting a prominent role of CB1 signalling in modulating these processes. Downregulation of its expression either pharmacological blockade or genetic disruption is associated with increased anxiety-, and depressive-like behaviour, impaired extinction of aversive memories and increased stress sensitivity and following onset of neuroinflammation.

Various approaches are used in order to use microglia cells as model cell lines. For example, US6780641B2 describes the use of amphotropic replication-incompetent retroviral vector encoding *v-myc* oncogene transcribed from mouse leukemia virus LTR plus neomycin-resistant gene transcribed from an internal SV40 early promote to generate immortalized human microglia cell lines. WO2021026294A1 disclosure features CX3CR1 hemizygous and/or homozygous defective cells and methods of using such cells for the treatment of a metabolic or neurological disorder. This approach focuses on inserting the exogenous nucleic acid molecule into the genome of the cell at the CX3CR1 gene locus to target metabolic or neurological diseases. The reconstitution of microglial cells after transplantation of CX3CR1^{+/Grp} hematopoietic cells.

In view of the above, a need for controlled microglia cell cultures that could be used as cell models for researchers studying these cells in various contexts, such as neuroinflammation, neurodegenerative diseases, and brain development remains.

### SUMMARY OF THE INVENTION

The present invention relates to a method for immortalization of mouse brain isolated microglia with subsequent knockout of endocannabinoid receptors to provide a reliable model for developing strategies to treat neurodegenerative diseases. Advantageously, the present invention provides a method that generates long-term microglial cells.

The present invention provides a novel method for obtaining immortalized microglial endocannabinoid receptor gene knockout cells. The method comprises the following steps: a) obtaining primary microglial cells; b) immortalization of microglial cells; c) producing endocannabinoid receptor gene knockout in the cells. The present disclosure further provides immortalized microglial endocannabinoid receptor gene knockout cells obtained by the method.

### BRIEF DESCRIPTION OF FIGURES

The drawings are provided as a reference to possible embodiments and are not intended to limit the scope of the invention. Neither of the drawings nor the graphs presented herein should be construed as limiting the scope of the invention, but merely as an example of a possible embodiment.
Fig. 1 shows the analysis of the homogeneity of primary microglial cell culture: immunocytochemistry was performed with anti-lba1 (a microglia-specific calcium-binding protein) antibodies. (A) shows Iba1-positive microglia cell culture; (B) shows a quantitative analysis of Iba1-positive microglia compared to total cell number. Data are expressed as a mean ± standard deviation. The dots represent the number of Iba1-expressing cells. Abbreviations: DAPI - nuclear dye; Iba1 is a microglia-specific marker.
Fig. 2 shows a comparison of the expression of β-galactosidase, an enzyme related to cell ageing, in primary microglial cells and the BV-2 cell line (shown as dark grey cells): the primary cells are about 16 times more prone to undergo cell senescence (quantitative analysis) 7 days after isolation.
Fig. 3 shows microglia cells 7, 4 and 17 days after transduction with lentiviruses containing pCW57.1-SV40 plasmid - cells rapidly proliferate and tend to reach confluence. (A) 4X magnification; (B) 10X magnification.
Fig. 4 shows Western blot analysis of CB1 expression in *Cnr1* knockout microglia. Tubulin expression serves as a control for expression. K denotes the control protein for CB1 and tubulin, respectively.
Fig. 5 shows the phagocytic activity of microglia cells. The arrows indicate that the cells are more inclined to engulf latex beads after stimulation with LPS.

### DETAILED DESCRIPTION OF THE INVENTION

The present disclosure provides a method for generation of immortalized microglial endocannabinoid receptor gene knockout cells, as well as immortalized microglial endocannabinoid receptor gene knockout cells obtained by said method.

According to the present disclosure, a method for generation of immortalized microglial endocannabinoid receptor gene knockout cells comprising the steps: a) obtaining primary microglial cells, b) immortalizing the microglial cells, and c) producing endocannabinoid receptor gene knockout in the cells is provided.

In some embodiments, the microglial cells that are generated by the presently disclosed method are mammalian microglial cells. In some embodiments, the microglial cells are rodent microglial cells. In preferred embodiments, the microglial cells are mouse microglial cells.

In some aspects, in the presently disclosed method for generation of immortalized microglial endocannabinoid receptor gene knockout cells, the primary microglial cells are immortalized through viral-mediated induction of the large T-antigen. In preferred embodiments, the viral-mediated induction of the large T-antigen is made by using SV40 large T antigen.

In some aspects, in the presently disclosed method for generation of immortalized microglial endocannabinoid receptor gene knockout cells, the endocannabinoid receptor gene knockout is produced using CRISPR-Cas system. In some embodiments, the CRISPR-Cas system is CRISPR-Cas9 nickase system.

In some embodiments, the endocannabinoid receptor gene that is knocked out in the immortalized microglial cells according to the present invention, is *Cnr1.* In other embodiments, the endocannabinoid receptor gene that is knocked out in the immortalized microglial cells according to the present invention, is *Cnr2.*

The present disclosure further provides an immortalized microglial endocannabinoid receptor gene knockout cell. Preferably, the immortalized microglial endocannabinoid receptor gene knockout cell is obtained by the method according to the present disclosure. In some embodiments, the immortalized microglial endocannabinoid receptor gene knockout cell is obtained from mouse primary microglial cell. In some embodiments, the immortalized microglial endocannabinoid receptor gene knockout cell is an immortalized microglial *Cnr1* knockout cell. In other embodiments, the immortalized microglial endocannabinoid receptor gene knockout cell is an immortalized microglial *Cnr2* knockout cell.

The method of the present invention advantageously combines the steps of a) obtaining primary microglial cells, b) immortalizing the microglial cells, and c) producing endocannabinoid receptor gene knockout in the cells.

Various methods may be suitable for obtaining primary microglial cells for use in the method for generation of immortalized microglial endocannabinoid receptor gene knockout cells of the present disclosure.

As used herein, the term "cell immortalization" refers to the increase in proliferative potential of cells beyond the Hayflick limit and does not necessarily imply neoplastic transformation. Immortalized cells differ from cultured primary cells in that they have a genetic modification that renders them immortal. There are various methods of rendering mammalian cells immortal under culture conditions, such as the use of viral genes, such as the Simian Virus 40 (SV40) T antigen, overexpression of hTERT, co-expression of the catalytic subunit of hTERT with either p53 or RB siRNA, overexpression of Ras or Myc T58A, etc.

As used herein, the term "gene knockout" refers to a mutation of the DNA that permanently stops the expression of the gene. This is possible in all types of cells and organisms using specific genetic approaches. Currently, besides Cre-LoxP, conventional homologous recombination, the fastest and most direct approach to achieve a specific gene knockout is the use of CRISPR genome editing tools.

As used herein, the term "microglia" refers to the resident immune cells of the brain that constantly monitor the cerebral microenvironment to respond to pathogens and damage. Microglia are highly ramified cells and their processes are very active and dynamic even under non-pathological conditions.

### EXAMPLES

### Microglia cell isolation from mice brain

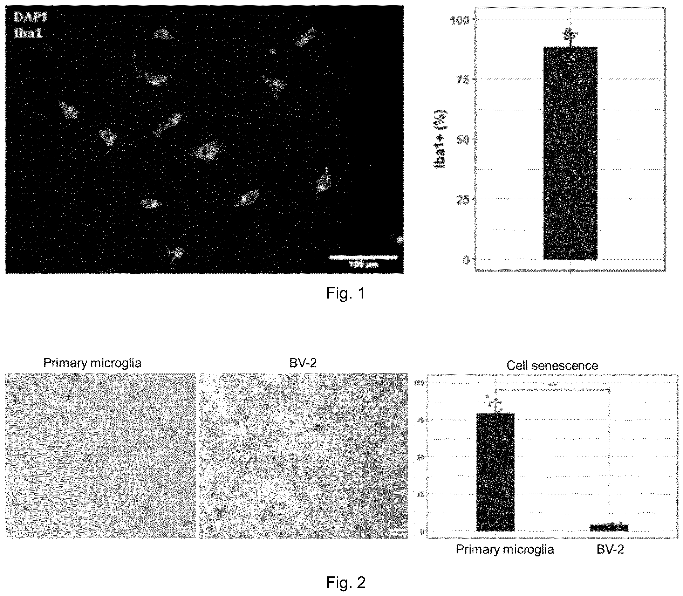

Animal care and experiments were performed in accordance with the requirements of the 2010/63/EU directive. Animals were housed under controlled conditions (22 °C ± 1, 40% humidity; food and water provided *ad libidum*) under the supervision of a veterinarian. Cell isolation was performed from 12-14-month-old male and female C57BL/6 subline mice. Cervical dislocation and decapitation were performed. Removed brains were transported in transfer buffer (97% DMEM/F-12; 3% 100× penicillin/streptomycin) (Gibco I Thermo Fisher Scientific). In a laminar hood, brains were transferred to a Petri dish with 6 ml of tissue digestion solution (2 mg/ml collagenase A; 28 U/ml DNase I; 0.5% 1M HEPES; 5% of heat inactivated FBS and HBSS (without Ca²⁺ and Mg²⁺)) (Gibco I Thermo Fisher Scientific) and minced with a scalpel. Minced tissues were collected and incubated in an incubator for 15 min 37 °C with gentle agitation every 5 minutes. After incubation, the sample was pipetted with Pasteur pipettes of different diameters (L/M/S) until resistance was no longer felt. The sample was incubated for 15 min 37 °C with gentle agitation every 5 minutes. The sample was filtered through a 70 µm pore size filter. The following steps were performed on ice. The cell sample was supplemented with HBSS (with Ca²⁺ and Mg²⁺) at a volume of 14 ml, centrifuged for 10 min 300 g at 4 °C at maximum acceleration and brake. The supernatant was discarded, the sediment was slowly suspended in 2 ml of 4 °C PBS and 1200 µL of Debris removal solution (Miltenyi Biotec). Then 4 °C PBS was added to the sample very slowly to 14 ml. Centrifuged for 10 min 3000 g at 4 °C at maximum acceleration and maximum brake. After centrifugation, the upper layer of PBS and the middle layer of myelin were removed. The sample was supplemented with PBS to a volume of 14 ml, centrifuged for 10 min 1000 g at 4 °C at maximum acceleration and maximum brake. After removing the supernatant, the cells were suspended in 5 ml of microglial culture medium (89% DMEM/F-12; 10% heat-inactivated FBS; 1% 100× penicillin/streptomycin) and transferred to a 12-well plate for cultivation. After 2-4 hours, the medium from the cells was changed to fresh microglial culture medium with 10 ng/ml M-CSF and GM-CSF (Sigma-Aldrich). Cells were grown at 37 °C in a 5% CO₂ atmosphere. The purity of the isolated microglia culture was confirmed microscopically using anti-Iba1 antibodies (Fig. 1). More than 90 % of culture were Iba1-positive cells.

Microglia senescence was evaluated by Senescence Cells Histochemical Staining Kit (Merck) which is based on the β-galactosidase activity at pH 6. Lysosomal β-galactosidase cleaves β-D-galactose residues into β-D-galactosides. Detectable β-galactosidase activity is the most commonly used marker of ageing or senescent cells, whether in culture or in mammalian tissue. Specifically, the enzyme β-galactosidase hydrolyses 5-bromo-4-chloro-3-indoyl-β-d-galactopyranoside, a colorless, soluble compound consisting of galactose bound to an indole. This reaction releases a deep blue, insoluble product on the cell culture or in the tissue (de Mera-Rodríguez et al., 2021) (in Figure 2, the reaction product can be seen as a dark grey dots in the senescent cells). β-galactosidase activity is detectable in senescent cells, but not in quiescent, immortal, or tumor cells.

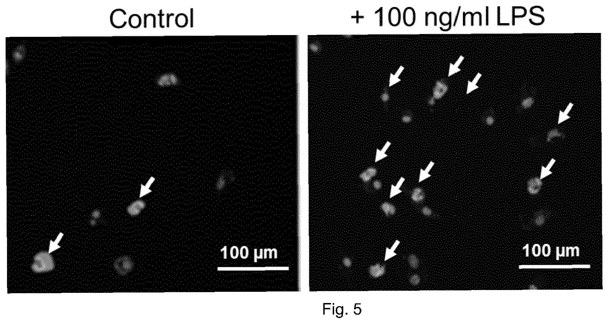

Phagocytic activity was evaluated microscopically by uptake of the 1 µM size carboxylate-modified polystyrene latex beads, fluorescent yellow-green (Merck) (Fig. 5).

### Immortalization of microglial cells

In order to immortalize microglial cells, first specific PacI restriction endonuclease cleavage sites at the 5' and 3' ends of the MAT2A gene were introduced into the commercial pCW57.1-MAT2A plasmid (Addgene). Since the 4545 bp theoretical product of plasmid hydrolysis after introducing a mutation at the 3' end of the MAT2A gene was visually shorter than the 4545 bp length, the mutated part of the plasmid with PacI restriction endonuclease hydrolysis regions were cloned back into commercial pCW57.1 -MAT2A plasmid. After analytical hydrolysis of plasmids using Xhol, Kpnl, and PacI restriction endonucleases, it was shown that plasmids have their expected integrity according to the hydrolysis pattern. Gel electrophoresis results were compared to hydrolysis patterns obtained in silico, and band sizes corresponded with theoretical sizes... After amplification of SV40 large T antigen, cMYC, hTERT genes from plasmids, they were ligated into the pCW57.1 mutated plasmid. After selecting the positive clones and confirming the integrity of the plasmids, the plasmids were further used in the immortalization of microglia. After the plasmids were constructed, the plasmids were aimed to be introduced into microglial cells of aged mice using lentiviral transduction. The transduction of pCW57.1-c-Myc and pCW57.1-hTERT did not induce microglial proliferation. In contrast, pCW57.1-SV40 showed to be efficient for microglia immortalization, the procedure of transduction is described below.

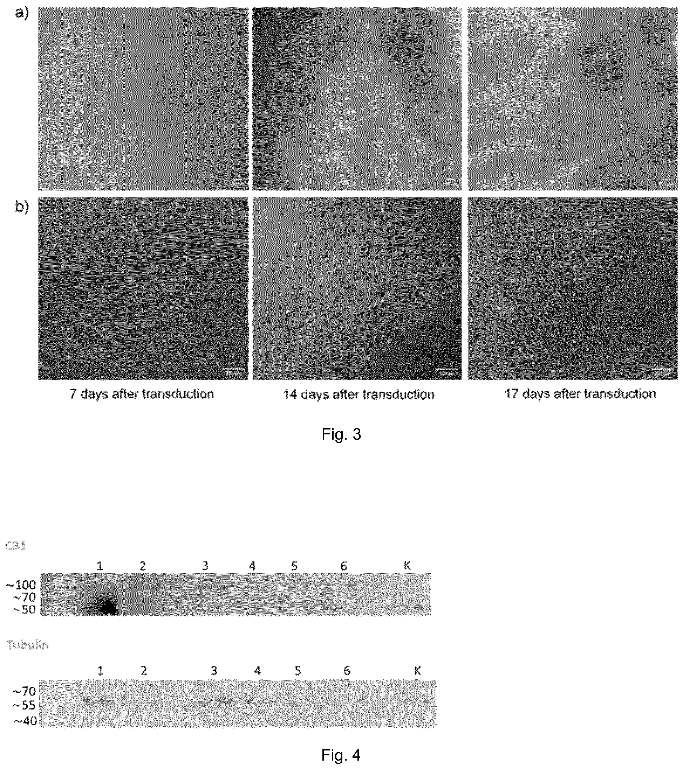

Lentivirus packaging HEK293T (Thermo Fisher Scientific) cells were seeded in a 6-well plate at a density of 2 × 10⁴ cells/well in culture medium (89% DMEM; 10% FBS; 1% 100 × penicillin/streptomycin). After 24 hours incubation cells were transfected with plasmid vectors: pCW57.1-SV40:pCMV-dR8.2 dvpr:pCMV-VSV-G (Addgene) in a 1:1:0.2 ratio (2500 ng per well ) using Lipofectamine 3000 reagent, according to the manufacturer's recommendations. The transfection mixture was dropped onto HEK 293T cells and incubated for 6 hours. The medium was changed to fresh culture medium and incubated for 48 hours. Microglial cells for transduction were seeded into a well of a 6-well plate separately for each animal for microglial cells from aged mice; neonatal microglial cells were plated at a density of 10 × 10⁴ cells/well. Plates were incubated for 24 hours. The lentiviral medium generated after transfection of HEK 293T cells was collected and filtered through a sterile 0.45 µm pore filter, diluted 1:1 with culture medium, and then transferred onto target cells. To improve transduction efficiency, 8 µg/ml polybrene (Merck) was added to the medium. The cells were placed in an incubator. After 24 hours the lentivirus medium was replaced with fresh culture medium. Cell morphology was analyzed using light microscope (Fig. 3).

### Knockout of endocannabinoid receptor gene in microglial cells

Cas9(D10A) nickase is encoded in the DNA sequence of AIO-puro plasmid, which forms a single-stranded DNA break in the complementary sequence of the DNA strand of the gRNA, so in the case of this system, two gRNAs are used. Using the open access platform Benchling^{©}, 3 gRNA pairs were selected according to the following criteria: 1. The PAM regions are upstream of the primary gRNA and downstream of the secondary gRNA, reading from the 5' end; 2. The lower the probability of non-target interaction and the higher the probability of targeted interaction; 3. Location of single-strand breaks as close as possible to the beginning of the protein coding sequence. Since the results obtained *in silico* do not always correspond to the results of *in vitro* studies, in order to test the gRNA and subsequently knockout the *Cnr1* gene in immortalized mouse microglial cells, AIO-puro plasmids containing 3 different gRNA pairs for both genes were constructed. After inserting the first of the two gRNAs, colony PCR and DNA electrophoresis were performed. Four colonies of competent *E.Coli* DH5α strain bacteria transformed with constructed plasmids were tested. After correct ligation, a 274 bp PCR fragment were visible in the gel. Plasmids were isolated from overnight cultures of these clones and inserted with the corresponding second gRNA. After correct ligation, PCR fragments of 274 and 744 bp were visible in the gel.

Construction of pCAG-EGxxFP-Cnr1 plasmid was performed in order to confirm that gRNA binds to the target sequences and generates two single-stranded breaks there. Mouse Cnr1 (524 bp long) gene fragments were amplified from tail biopsy by PCR method, purified, restricted, purified again after restriction and ligated into pCAG-EGxxFP plasmid. Universal Control Primer Mix of the Phire Tissue Direct PCR Kit (Thermo Fisher Scientific), which is complementary to a fragment of the conservative non-coding region upstream of the SOX21 gene in the mammalian genome, was also used to amplify the 237 bp PCR product. Competent *E.Coli* DH5α strain bacteria were ligatetransformed and restriction analysis was performed to select suitable clones. After correct ligation, pCAG-EGxxFP-Cnr1 is restricted to 3695 bp, 1972 bp and 1208 bp long fragments, which should be visible in the gel.

After the plasmids were constructed, gRNA efficiency was further tested in HEK297T cells. After cotransfecting cells with AIO-Puro-Cnr1-gRNA and pCAG-EGxxFP-Cnr1, Cas9(D10A) nickase should form two single-strand breaks in pCAG-EGxxFP-Cnr1 plasmid between the EGFP coding sequence, where the target *Cnr1* gene fragment is inserted and its removal should lead to the synthesis of functional enhanced green fluorescent protein (EGFP).

Cells were transfected with the AIO-puro-gRNA plasmid using LipfectamineTM 3000 Transfection Reagent (Thermo Fischer Scientific) according to the manufacturer's instructions. Two days after transfection, selection was performed with puromycin. Cells were grown to confluence and then lysed for protein electrophoresis and Western blot to verify that knock-out was successful.

An analogous strategy was developed and successfully applied for the *Cnr2* gene.

**Table of gRNA sequences used in knockout experiments:**

| | | |
|---|---|---|
| mCNR1-sgRNA1-1 | ACCGGCCCACGTAGAGGAGGTCTG | AAACCAGACCTCCTCTACGTGGGC |
| mCNR1-sgRNA1-2 | ACCGATTCAGTACGAAGATATCAA | AAACTTGATATCTTCGTACTGAAT |
| mCnr1-sgRNA2-1 | ACCGAAGTTAGAGGGAATTTCTGT | AAACACAGAAATTCCCTCTAACTT |
| mCnr1-sgRNA2-2 | ACCGTCCCTTCCAAGAAAAGATGA | AAACTCATCTTTTCTTGGAAGGGAC |
| mCnr1-sgRNA3-1 | ACCGTTGGAAGGGACTACCCCTGA | AAACTCAGGGGTAGTCCCTTCCAA |
| mCnr1-sgRNA3-2 | ACCGGGCAGGAGACAACTCCCCGT | AAACACGGGGAGTTGTCTCCTGCC |
| mCnr2-sgRNA1-1 | ACCGGGATCATGTACTCCTTCATG | AAACCATGAAGGAGTACATGATCC |

### References

Alberts, B., Johnson, A., Lewis, J., Raff, M., Roberts, K., Walter. P. Molecular Biology of the Cell. 4th edition. New York: Garland Science; 2002. ISBN-10: 0-8153-3218-1ISBN-10: 0-8153-4072-9 Cserép, C., Pósfai, B., Lénárt, N., Fekete, R., László, Z.I., Lele, Z., Orsolits, B., Molnár, G., Heindl, S., Schwarcz, A.D., Ujvári, K., Környei, Z., Tóth, K., Szabadits, E., Sperlágh, B., Baranyi, M., Csiba, L., Hortobágyi, T., Maglóczky, Z., Martinecz, B., Szabó, G., Erd6lyi, F., Szipőcs, R., Tamkun, M.M., Gesierich, B., Duering, M., Katona, I., Liesz, A., Tamás, G., Dénes, A., Microglia monitor and protect neuronal function through specialized somatic purinergic junctions. Science 2020, 367, 528-537.

De Meij, J., Alfanek, Z., Morel, L., Decoeur, F., Leyrolle, Q., Picard, K., Carrier, M., Aubert, A., Séré, A., Lucas, C., Laforest, G., Helbling, J-Ch., Tremblay, M-E., Cota, D., Moisan, M-P., Marsicano, G., Layé, S., Nadjar, A. Microglial Cannabinoid Type 1 Receptor Regulates Brain Inflammation in a Sex-Specific Manner. Cannabis Cannabinoid Res 2021, 6(6):488-507. doi: 10.1089/can.2020.0170.

de Mera-Rodríguez, J. A., Álvarez-Hernán, G., Gañán, Y., Martín-Partido, G., Rodríguez-León, J., Francisco-Morcillo, J. Is Senescence-Associated β-Galactosidase a Reliable in vivo Marker of Cellular Senescence During Embryonic Development? Front. Cell Dev. Biol., 2021, Sec. Cell Death and Survival, 9 - 2021. doi.org/10.3389/fcell.2021.623175

Lee, J.-W., Lee, I.-H., limura, T., Kong, S.W., 2021. Two macrophages, osteoclasts and microglia: from development to pleiotropy. Bone Res 9, 11.

Timmerman, R., Burm, S.M., Bajramovic, J.J. An Overview of in vitro Methods to Study Microglia. Front Cell Neurosci. 2018; 12: 242. doi: 10.3389/fncel.2018.00242

Young, A. P., Denova-Wright, E. M. The Dynamic Role of Microglia and the Endocannabinoid System in Neuroinflammation. Front Pharmacol. 2021; 12: 806417. doi: 10.3389/fphar.2021.806417

## Claims

1. A method for generation of immortalized microglial endocannabinoid receptor gene knockout cells, comprising the steps:
a) obtaining primary microglial cells;
b) Immortalizing the microglial cells, and
c) Producing endocannabinoid receptor gene knockout in the cells.

2. The method according to claim 1, wherein the microglial cells are mammalian microglial cells.

3. The method according to claim 2, wherein the microglial cells are rodent microglial cells.

4. The method according to claim 3, wherein the microglial cells are mouse microglial cells.

5. The method according to any one of claims 1 to 4, wherein the primary microglial cells are immortalized through viral-mediated induction of the large T-antigen.

6. The method according to claim 5, wherein the viral-mediated induction of the large T-antigen is made by using SV40 large T antigen.

7. The method according to any one of claims 1 to 6, wherein the endocannabinoid receptor gene knockout is produced using CRISPR-Cas system.

8. The method according to claim 7, wherein the CRISPR-Cas system is CRISPR-Cas9 nickase system.

9. The method according to any one of claims 1 to 8, wherein the endocannabinoid receptor gene is Cnr1 or Cnr2.

10. An immortalized microglial endocannabinoid receptor gene knockout cell obtained by the method according to any one of the claims from 1 to 9.

11. The immortalized microglial endocannabinoid receptor gene knockout cell according to claim 10, wherein the endocannabinoid receptor gene is *Cnr1.*

12. The immortalized microglial endocannabinoid receptor gene knockout cell according to claim 10, wherein the endocannabinoid receptor gene is *Cnr2.*
